Europäisches Patentamt

European Patent Office

Office européen des brevets

(19)

(11) Publication number: **0 010 526**
**B1**

# EUROPEAN PATENT SPECIFICATION

(12)

(45) Date of publication of patent specification: **03.08.83**  (51) Int. Cl.³: **G 01 N 33/86**

(21) Application number: **79830026.5**

(22) Date of filing: **31.08.79**

(54) Method and apparatus for investigating haemocoagulation or aggregation of platelets.

(30) Priority: **07.09.78 IT 8345678**

(43) Date of publication of application:
**30.04.80 Bulletin 80/9**

(45) Publication of the grant of the patent:
**03.08.83 Bulletin 83/31**

(84) Designated Contracting States:
**AT BE CH DE FR GB LU NL SE**

(56) References cited:
**DE - A - 1 498 574**
**DE - A - 1 598 788**
**DE - A - 2 401 084**
**DE - A - 2 408 214**
**DE - A - 2 413 285**
**DE - B - 2 058 973**
**DE - B - 2 258 337**
**FR - A - 2 301 011**
**GB - A - 1 270 416**
**GB - A - 1 302 396**
**GB - A - 1 337 344**
**US - A - 3 302 452**

(73) Proprietor: **SALUS ISTITUTO DIAGNOSTICO DI CROCE' DR.FRANCESCO & C. s.a.s.**
**n.71 Viale Ungheria**
**I-33100 Udine (IT)**

(72) Inventor: **Breda, Enzo**
**n. 34 Via Paradattimis**
**I-33100 Udine (IT)**
Inventor: **Ciotti, Alfredo**
**n. 60 Piazzale Chiavris**
**I-33100 Udine (IT)**

(74) Representative: **Petraz, Gilberto**
**G.L.P. S.a.s. di Gilberto Petraz P.le Cavedalis 6/2**
**I-33100 Udine (IT)**

(56) References cited:
**US - A - 3 307 392**
**US - A - 3 450 501**
**US - A - 3 527 569**
**US - A - 3 658 480**

Courier Press, Leamington Spa, England.

Method and apparatus for investigating haemocoagulation or aggregation of platelets

The invention relates to a method and apparatus for investigating the phenomenon of coagulation of blood or aggregation of blood platelets.

It is well known that several types of apparata are used in the art to analyze the phenomena of blood coagulation and aggregation of blood platelets. In particular two types of apparata are hitherto used for investigating these two phenomena.

For the blood coagulation few types of apparata are used to detect the development of the reaction:

— one apparatus is of electro-mechanical type and is based on the fact that the plasma, while it is coagulated, blocks the regular agitation of a magnetic ball which on the absence of agitation signals the time taken by the blood to coagulate;

— another apparatus is of the electro-optical type and is based upon the light absorption which increases gradually as the coagulation proceeds so that in addition to a preestablished minimum threshold, it is possible to establish the periods of the time required for the coagulation;

— one other type of apparatus and the corresponding method is disclosed in GB 1,270,416 which is based on photoelectrically detecting changes in the electric impulses resulting from an increase in the viscosity of a sample mixed with a reagent contained in a rocking container whose oscillation are compared with the frequency of electric impulses.

When the blood clotting is formed the electric impulses cease and the time measured from the addition of the reagent is recorded to give prothrombin time.

The drawback of this method is that the time obtained is not sufficiently accurate since the clotting is gradual and not sudden which means that clotting is not immediately detected.

For the platelet aggregation some instruments are used which give an electro-optical measurement of the light absorbed by the platelets being aggregated and by the platelets which are not aggregated as a function of the percentage of the aggregation.

One drawback of these apparata resides in the fact that they give the two values separately and therefore the tests must be carried out on two different apparata with the result that errors are likely to occur.

Another drawback of the apparata known in the art relates to the analysis of blood coagulation and specifically resides in the fact that an increase in the opacity or optical density is detected while the thresholds of opacity in percent remain more or less fixed, the latter arresting the timer from the start of the reaction.

The result is that there is no uniformity in the detection with respect to a minimum or a maximum relative to the optical density or opacity manifested during the reaction.

Furthermore, due to the fact that the percent thresholds of a pre-established opacity remain fixed from the start of the analysis, some drawbacks may arise due to the intrinsic or blank opacity of the plasma under examination, which intrinsic opacity may vary from individual to individual.

The method disclosed in FR 2301011 represents an attempt to eliminate the effect of the intrinsic opacity by subtracting the initial electric signal corresponding to the blank opacity from the signal corresponding to an electric signal proportional to the opacity after the reaction starts.

This has the effect of grounding the opacity curve which is then printed on moving tape.

The test time is measured by marking manually on the tape the initial point of the clotting and calculating it as a function of the tape speed.

This procedure is obviously very slow and of doubtful accuracy due to the presence of the initial transient response of the reaction which makes it difficult to pin-point the actual start point of the clotting or fibrin formation.

One may also point out that certain attempts have been made as for example in DE—A1—2413285 to convert the signal proportional to the opacity to numerical or alphanumerical signals without however improving the accuracy of the test.

The object of the present invention is to eliminate the drawbacks mentioned hereinabove.

Another object of the present invention is to provide a single apparatus which enables the carrying out of two types of investigations that permit: a substantial saving in the space being used; a substantial saving in the time required; a reduction in the possibility of errors being caused by the exchange of the samples since no transfer of samples is required.

The present invention proposes a method for investigating haemocoagulation or platelet aggregation based on producing an electric signal proportional to the optical density of a stirred blood sample mixed with reagents, com-prising the step of:

— inserting a sample of blood in the test tube, stirring it, detecting the optical density and setting to zero of the electrical signal proportional to the optical density;

and characterized by further comprising the steps of:

— presetting a threshold level of the optical density;

— adding of reagents to the blood sample, and automatic starting of a timer triggered by the initial transient variation of the optical density due to the outset of the reaction between the blood and the reagent;

— automatic second setting to zero and restarting of the timer and resetting to zero of said electrical signal after a first time interval which is predetermined in dependence on said types of investigation;

— automatic stopping of the timer after a second time interval, when said electrical signal has reached a level corresponding to the preset threshold level of the optical density; and

— recording the value of said second time interval.

The present invention also discloses an apparatus for performing the method of claim 1 for studying haemocoagulation or platelet aggregation comprising means for receiving a blood sample and reagents, means for stirring the mixture; means for detecting the optical density of the sample and mixture capable of generating an electrical signal proportional to said optical density, a timer as well as control means and programming means characterized as disclosed in claim 2.

According to an embodiment of the present invention in the apparatus said timer is connected to a printer.

According to another embodiment of the invention said amplifying setting means are connected to a recorder.

The method and apparatus according to the present invention enable the investigation of both platelet aggregation and of blood coagulation which is effected by means of an automatic setting of an electrical signal at the optimum point of the reaction itself while the timer is triggered by the first disturbances that occur in the optical density at the start of the reaction when the reagents are mixed.

This setting, which corresponds to blanking, depends on the type of blood coagulation investigation and is determined on the basis of a large amount of experimental data collected from tests.

As an example, in the case of a test which lasts about 10 seconds, the optimum experimental setting, according to which the instrument is calibrated for that particular investigation, is carried out during the sixth second and so on for other tests of different duration.

Furthermore, the apparatus according to the present invention is provided with means to select the type of investigation for instance a six-position selector that makes it possible to obtain the following:

1) prothrombin time;
2) partial thromboplastin time (ptt);
3) calcification time or Howell time;
4) fibrinogen determined by means of measuring the time of coagulation with conventional reagents;
5) determination of troimbodensitogram and fibrinolytic activity or any kind of lytic phenomena i.e. erithrocytes;
6) investigating platelet aggregation by means of magnetic-mechanical stirring of the plasma with the aid of the addition of suitable reagents.

The above listed investigations are carried out by the same apparatus with the aid of suitable control means acting on the above-mentioned means for selecting the type of investigation and which automatically establishes also the various settings for each investigation.

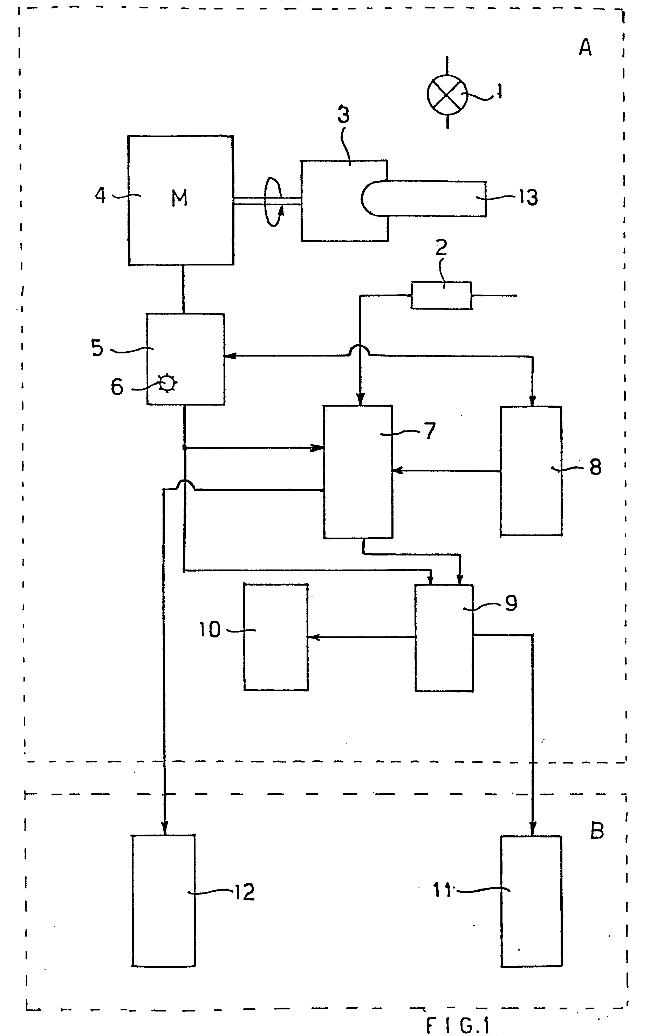

The features mentioned hereinabove will be better understood and other features will become apparent from the following detailed description of an embodiment of the invention with reference to the attached Fig. 1 which shows a schematic block diagram of the apparatus according to the present invention.

In Fig. 1 it is shown that the apparatus designated by the letter A, may be connected to other auxiliary devices which are designated by the letter B, and which are easily available on the market.

These auxiliary devices B are not described in detail because they are in existence in the market and are well known to people skilled in the art.

The apparatus of the present invention designated by the letter A, consists essentially of a lamp which has a constant light intensity source designated by numeral 1, an electro-photodetector designated by numeral 2, a support for a test tube designated by numeral 3 provided with a thermostat to maintain a constant test temperature, generally about 37°C, a stirring device 4 which is electrically connected to and controlled by a programmer 5, the latter being provided with a dial designated by numeral 6 which may occupy anyone of six positions.

The apparatus is further provided with an amplifier zero-setting element 7 which is connected to the photodetector 2 and to the programmer 5 as well as to an analogical memory 8 which is also linked to the programmer 5.

The apparatus comprises a device for measuring time or a timer 9 which is connected to the amplifier setting element 7 and with the programmer 5 and a digital monitor 10 which is also connected to the timer 9.

The timer 9 is also connected to a printing device 11 and the amplifier setting element 7 is in its turn connected to a recorder 12.

The test tube 13 containing the blood sample under test, is placed on the support 3; the test process being carried out as described hereinabove.

It is obvious that the details of the embodiment can be varied without altering the features of the apparatus according to the present invention, which features are substantially defined in the claims attached hereto.

## Claims

1. A method for investigating haemocoagulation or platelet aggregation based on producing an electric signal proportional to the optical density of a stirred blood sample mixed with reagents, comprising the steps of:

— inserting a sample of blood in a test tube, stirring it, detecting the optical density and setting to zero of the electrical signal proportional to the optical density;

and characterized by further comprising the steps of:

— presetting a threshold level of the optical density;
— adding of reagents to the blood sample, and automatic starting of a timer triggered by the initial transient variation of the optical density due to the outset of the reaction between the blood and the reagent;
— automatic second setting to zero and restarting of the timer and resetting to zero of said electrical signal after a first time interval which is predetermined in dependence on said types of investigation;
— automatic stopping of the timer after a second time interval, when said electrical signal has reached a level corresponding to the preset threshold level of the optical density; and
— recording the value of said second time interval.

2. Apparatus for performing the method of Claim 1 for studying haemocoagulation or platelet aggregation comprising means (13) for receiving a blood sample and reagents, means (4) for stirring the mixture; means (2) for detecting the optical density of the sample and mixture capable of generating an electrical signal proportional to said optical density, a timer (9) as well as control means and programming means (5, 6) characterized in that

— amplifying and setting means (7) receiving the said electric signal is provided;
— the control and programming means (5, 6) is adapted to act on the timer (9) and on the said amplifying and setting means (7) in such a manner that the timer (9) is reset and started and the electrical signal is set to zero at the outset of the reaction and again after a first time interval which is predetermined for each of the said types of investigations and in such a manner that the timer (9) is stopped after a second time interval, the beginning of which is the end of the first time interval and the end of which is determined by the surpassing of a threshold level by the electrical signal.

3. Apparatus as in Claim 2 characterized by the fact that said timer (9) is connected to a printer.
4. Apparatus as in Claim 2 characterized by the fact that said amplifying setting means (7) are connected to a recorder (12).

## Patentansprüche

1. Verfahren zur Analyse der Blutgerinnung oder des Thrombozytenaggregats, das sich auf die Bildung eines elektrischen der optischen Dichte einer geschüttelten und mit Reaktionsmitteln gemischten Blutprobe proportionalen Signals stützt und das folgende Phasen miteinbezieht:

— Einführung einer Blutprobe in ein Reagenzglas, Schütteln derselben, Erhebung der optischen Dichte und Nulleinstellung des elektrischen der optischen Dichte proportionalen Signals;

und dadurch gekennzeichnet, daß es außerdem folgenden Phasen miteinbezieht:

— Vorausbestimmung eines Schwellwertes der optischen Dichte;
— Beimengung von Reaktionsmitteln der Blutprobe und automatischer Start eines Timers, das von der vorübergehenden Anfangsänderung der optischen Dichte geregelt ist, die vom Beginn der Reaktion zwischen Blut und Reaktionsmittel verursacht ist;
— Zweite automatische Nulleinstellung und Start des Timers und Wiedernulleinstellung des genannten elektrischen Signals nach einem ersten Zeitraum, der infolge der genannten Analysentypen vorausbestimmt ist;
— Automatisches Stop des Timers nach einem zweiten Zeitraum, wenn das genannte elektrische Signal einen dem vorgewählten Schwellwert der optischen Dichte entsprechenden Wert erreicht hat; und
— Einstellung des Wertes im genannten zweiten Zeitraum.

2. Vorrichtung zur Durchführung des Verfahrens gemäß Anspruch 1 zum Studium der Blutgerinnung oder des Thrombozytenaggregats, die Mittel (13) zum Empfang einer Blutprobe und Reaktionsmittel, Mittel (4) zum Schütteln des Gemisches, Mittel (2) zur Erhebung der optischen Dichte der Probe und des Gemisches, die dazu geeignet sind, ein elektrisches der genannten optischen Dichte proportionales Signal zu bilden, ein Timer (9) und außerdem Kontrollmittel und Programmierungsmittel (5, 6) aufweist, dadurch gekennzeichnet, daß

— Verstärkungs- und Eichungsmittel (7) vorhanden sind, die das genannte elektrische Signan empfangen und daß
— die Kontroll- und Programmierungsmittel (5, 6) dazu geeignet sind, auf das Timer (9) und auf die genannten Verstärkungs- und Eichungsmittel so zu wirken, daß das Timer wieder geeicht und betätigt wird und das elektrische Signal zu Beginn der Reaktion und wieder nach einem ersten Zeitraum, der für jeden der genannten Analysentypen vorbestimmt ist, auf Null gestellt wird, so daß das Timer (9) nach einem zweiten Zeitraum zum Stillstand gebracht wird, dessen Beginn das Ende des ersten Zeitraumes ist und dessen Ende von der Überschreitung eines Schwellwertes von Seiten des elektrischen Signals bestimmt ist.

3. Vorrichtung gemäß Anspruch 2, dadurch gekennzeichnet, daß das genannte Timer (9) einem Drucker verbunden ist.

4. Vorrichtung gemäß Anspruch 2, dadurch gekennzeichnet, daß die genannten Eichungsverstärkungsmittel (7) mit einer Einstellungsvorrichtung (12) verbunden sind.

**Revendications**

1. Méthode pour l'étude de l'hémocoagulation ou de l'agrégation des plaquettes, basée sur la production d'un signal électrique proportionnel à la densité optique d'un échantillon de sang agité et mélangé à des réactifs, comprenant les étapes qui consistent:

— à introduire un échantillon de sang dans le tube à essais, à l'agiter, à détector la densité optique et à mettre à zéro le signal électrique proportionnel à la densité optique, et caractérisé par le fait qu'il comprend en outre les étapes qui consistent;
— à fixer à l'avance un niveau de seuil de la densité optique;
— à ajouter des réactifs à l'échantillon de sang et à mettre en marche automatiquement une minuterie déclenchée par la variation transitoire initiale de la densité optique, due au commencement de la réaction entre le sang et le réactif;

— à remettre automatiquement la minuterie à zéro une deuxième fois et à la remettre en marche et à remettre à zéro le signal électrique au bout d'un premier laps de temps qui est prédéterminé sous la dépendance des types d'études mentionnés;
— à arrêter automatiquement la minuterie au bout d'un deuxième laps de temps, quand le signal électrique a atteint un niveau correspondant au niveau de seuil de densité optique fixé à l'avance, et
— à enregistrer la valeur de ce deuxième laps de temps.

2. Appareil pour la mise en ouvre de la méthode selon la revendication 1, pour l'étude de l'hémocoagulation ou de l'agrégation des plaquettes, comprenant des moyens (13) servant à recevoir un échantillon de sang et des réactifs, des moyens (4) d'agitation du mélange, des moyens (2) de détection de la densité optique de l'échantillon et du mélange, capables d'engendrer un signal électrique proportionnel à cette densité optique, une minuterie (9) ainsi que des moyens de commande et des moyens de programmation (5, 6), caractérisé en ce qu'il comporte des moyens d'amplification et de réglage (7) recevant ledit signal électrique et en ce que les moyens de commande et de programmation (5, 6) sont conçus pour agir sur la minuterie (9) et sur les moyens d'amplification et de réglage (7) de telle sorte que la minuterie (9) est remise à zéro et mise en marche et que le signal électrique est mis à zéro au commencement de la réaction et à nouveau après un premier laps de temps qui est déterminé pour chacun des types d'études mentionnés et de telle sorte que la minuterie (9) s'arrête après un deuxième laps de temps dont le commencement est la fin du premier laps de temps et dont la fin est déterminée par le fait que le signal électrique dépasse un niveau de seuil.

3. Appareil selon la revendication 2, caractérisé par le fait que la minuterie (9) est reliée à une imprimante.

4. Appareil selon la revendication 2, caractérisé en ce que les moyens d'amplification et de réglage (7) sont reliés à un enregistreur (12).

A

B

F I G. 1